# FASCICULE DE BREVET EUROPEEN

(11) **EP 1 701 721 B1**
(45) Date de publication et mention de la délivrance du brevet: **09.12.2009**
(21) Numéro de dépôt: 05717659.6
(22) Date de dépôt: 07.01.2005
(51) Int. Cl.: A61K 31/415, A61P 43/00

(54) **UTILISATION DE LA PILOCARPINE POUR LE TRAITEMENT DES HYPOSIALIES**
VERWENDUNG VON PILOCARPIN FÜR DIE BEHANDLUNG VON HYPOPTYALISMUS
USE OF PILOCARPINE FOR HYPOPTYALISM TREATMENT

(30) Priorité: 09.01.2004 FR 0450050
(43) Date de publication de la demande: 20.09.2006
(73) Titulaire: Perovitch, Philippe, 33950 Lege Cap Ferret (FR); Maury, Marc, 33160 Saint Medard en Jalles (FR); Deymes, Jean, 33000 Bordeaux (FR)
(72) Inventeur: Perovitch, Philippe, 33950 Lege Cap Ferret (FR); Maury, Marc, 33160 Saint Medard en Jalles (FR); Deymes, Jean, 33000 Bordeaux (FR)
(74) Mandataire: Riege, Christian
(86) Numéro de dépôt international: PCT/FR2005/050012
(87) Numéro de publication internationale: WO 2005/067924

(56) Documents cités:
- WO-A-03/084515
- FR-A- 2 737 661
- US-A- 5 741 805
- DATABASE WPI Section Ch, Week 199404 Derwent Publications Ltd., London, GB; Class A96, AN 1994-034733 XP002292717 & WO 94/01108 A1 (THERATECH INC) 20 janvier 1994 (1994-01-20)
- ROSAS J ET AL: "Usefulness of basal and pilocarpine-stimulated salivary flow in primary Sjogren's syndrome. Correlation with clinical, immunological and histological features" RHEUMATOLOGY (OXFORD), vol. 41, no. 6, juin 2002 (2002-06), pages 670-675, XP002292716 ISSN: 1462-0324

## Description

La présente invention concerne une composition pour le traitement des hyposialies comprenant de la pilocarpine. Cette composition est à effets multiples directs, dans la sphère bucco pharyngée.

Les problèmes engendrés par les xérostomies sont nombreux et bien connus sans que des solutions satisfaisantes aient été apportées. La radiothérapie est un des traitements majeurs dans le cas de cancers de la sphère ORL qui conduit à des effets indésirables importants.

Les conséquences de cette sècheresse buccale sont notamment des troubles de la déglutition, de l' élocution pouvant conduire à une anorexie.

Or il convient d'analyser, préalablement à l'étude des solutions apportées par la composition selon la présente invention, les différents mécanismes mis en cause. La production salivaire est dépendante :
- des glandes salivaires principales : parotides, sous-maxillaires et sub-linguales, et
- des glandes salivaires accessoires qui sont disséminées sous toute la surface de la muqueuse buccale : labiales, jugales, palatines et vélaires, linguales et sub-linguales.

Ces deux étages sont eux-mêmes répartis en trois structures histologiques :
- glandes séreuses,
- glandes muqueuses, et
- glandes mixtes ou séro-muqueuses.

Les origines des hyposialies peuvent se décomposer en :
- hyposialies transitoires : état pré-ménopausique ou ménopausique, prises médicamenteuses tels que contraceptifs oraux, diurétiques, produits cardio-vasculaires, produits anti-inflammatoires, nnti-histaminiques, antidépresseurs, psychotropes, tranquillisants, anti-parkinsoniens, et
- hyposialies permanentes : évolution de maladies auto-immunes, syndrome de Goujerot-Sjögren qui affecte les glandes exocrines et muqueuses, des effets iatrogènes engendrés par un traitement aux irradiations thérapeutiques générant un déficit pouvant être néanmoins compensé par une stimulation thérapeutique.

Dans le cadre des traitements et des stimulations de ces mécanismes, on connaît la pilocarpine qui est un alcaloïde extrait des feuilles de *pilocarpus* de différentes *variétés jaborondi, pennatifolius, microphyllus*

Il apparaît que la pilocarpine de composition C₁₁H₁₆N₂O₂ est un liquide huileux, visqueux, sensible à la lumière et présentant un caractère amphiphile qui permet une dissolution à la fois dans l'eau et beaucoup plus facilement dans un solvant ou une composition de solvants organiques.

Ce principe actif, parasympathomimétique, stimule les sécrétions des glandes exocrines et donc les sécrétions des glandes salivaires et les sudoripares.

La teinture de *Jaborandi* est obsolète et n'a jamais reçu d'autorisation de mise sur le marché car il est impossible de déterminer avec exactitude la teneur en principe actif. De plus, comme pour toute teinture, on retrouvera la présence d'autres éléments ou composants ou substances exogènes qui n'ont fait l'objet d'aucune étude. La stabilité du produit et les modes de conservation sont également discutables.

Dans la pharmacopée actuelle, on connaît la pilocarpine comme principe actif sous forme orale et des médicaments sont actuellement commercialisés.

Dans l'administration de ces médicaments, il est à noter que la dose est de 5 mg de pilocarpine par comprimé à administrer 3 fois par jour, soit 15 mg de pilocarpine par jour, voire jusqu'à 30 mg par jour pour certains cas plus sévères. De fait, il a été constaté que cette prise de doses importantes conduite sur plusieurs semaines est à l'origine d'effets secondaires indésirables importants tels que des nausées, des hypersudations, des sensations vertigineuses, des bouffées de chaleur ou des asthénies.

Une conséquence première de la voie orale est le passage par la voie digestive qui conduit à une absorption et une biodisponibilité variables car le premier passage hépatique fait subir au principe actif une dégradation importante sous forme de métabolites n'ayant plus d'activités pharmacologiques. Il faut donc prévoir un surdosage pour que l'effet de la partie subsistante atteigne les zones d'activité buccales et sous-maxillaires.

Une autre conséquence est un effet retardé du principe actif puisque la première action de la pilocarpine ne s'exerce qu'avec un délai de 0,45 à 1 heure après la prise.

La composition incluant la pilocarpine selon la présente invention permet de limiter fortement les effets secondaires liés à une absorption par voie orale, améliore le rapport dose/effet et confère une forte amélioration de la biodisponibilité.

L'invention est maintenant décrite en détail suivant un mode de réalisation particulier, non limitatif , avec une illustration par des exemples de formes galéniques adaptées.

La composition selon la présente invention consiste à administrer la pilocarpine base ou sous forme de sels, chlorhydrate ou nitrate, sous une forme galénique spécifique d'un comprimé à usage sub-lingual à délitement lent lui permettant :
- d'accéder directement et instantanément aux récepteurs muscariniques des structures glandulaires sous-muqueuses,
- de se protéger et d'être stable vis-à-vis de la lumière, de la température et de l'oxydation,
- de se dissoudre et de se fixer localement en s'associant intimement aux tissus de la sphère bucco-pharyngée.

A cet effet, la pilocarpine est associée à au moins un polymère bioadhésif, au moins un tampon et au moins un lubrifiant.

A ces agents, on peut associer un adoucissant, une substance hydrophile et la formulation en comprimé peut prévoir des dimensions aptes à éviter la déglutition et le passage direct vers le tractus digestif.

Un exemple de formulation, pour un comprimé à usage sub-lingual est :
- pilocarpine base ou sous forme de sel : 2,5 mg
- stéarate de magnésium : 10,0 mg
- hydrogénophosphate sodique ou disodique : 90,0 mg
- méthocel K 100 : 50,0 mg
- PolyEthylèneGlycol 6 000 : 40,0 mg
- acide hyaluronique : 20,0 mg
- lysozyme chlorhydrate : 15,0 mg
- sorbitol qsp comprimé de 1000 mg : 772,5 mg

Le tampon pH à base d' hydrogénophosphate sodique ou disodique peut être remplacé par du carbonate ou du bicarbonate de sodium.

Le stéarate de magnésium est un lubrifiant adapté à la fabrication de comprimés et le PEG, en plus de ses propriétés lubrifiantes, est hydrophile conférant à la composition un effet adoucissant.

On peut aussi citer comme substances ayant les mêmes fonctionnalités que le PEG la famille des dérivés cellulosiques, des gommes ou d'autres polymères.

La famille des dérivés cellulosiques comprend notamment :
- carboxy-méthyl cellulose sodique,
- hydroxy-éthyl cellulose,
- hydroxy-propyl cellulose,
- hydroxy-propyl méthyl cellulose,
- hydroxy-propyl méthyl promellose,
- méthyl cellulose ou métolose, ou
- carboxy-méthyl cellulose.

Parmi les gommes, on peut citer :
- guar,
- xanthane, ou
- gomme arabique.

Des polymères adaptés pour cette application comprennent :
- acide alginique et dérivés,
- polymère carboxy-vinylique
- macrogols
- gélatine
- povidone, ou
- pectines.

Le chlorhydrate de lysozyme peut être ajouté de façon avantageuse afin de compenser le déficit en lysozyme salivaire physiologique dans le cas des hyposialies, Le dosage est à adapter mais une plage de proportions se situe entre 5 et 30 mg pour un comprimé de 1000 mg.

Un autre élément de meilleure mise en oeuvre de la pilocarpine dans l'application locale selon la présente invention est le recours à un substrat de masse qui est de faible poids moléculaire et de préférence hygroscopique.

On peut citer la famille des polyols, sorbitol, mannitol ou xylitol mais aussi le lactose, Ce caractère hygroscopique induit la mise en oeuvre de mouvements liquidiens depuis la cavité buccale vers ce substrat de masse facilitant la dissolution de la pilocarpine, la distribution et l'adhésion aux tissus muqueux.

La composition selon la présente invention conduit aux conséquences positives qui sont maintenant énumérées.

Grâce à la composition selon la présente invention, la pilocarpine provoque un premier effet direct car elle est immobilisée au contact direct des récepteurs muscariniques présents au sein des différentes structures glandulaires salivaires, glandes sous-muqueuses endo-buccales et sous-maxillaires.

La pilocarpine, ainsi maintenue au contact, est suffisamment bien dissoute et biodisponible pour être absorbée à travers les structures phospholipidiques des parois des cellules de l'épithélium stratifié de la muqueuse.

On note aussi du fait de la présence locale de la pilocarpine un accroissement du débit micro-vasculaire local avec sensation de chaleur et donc une augmentation de l'absorption locale tissulaire. L'effet est autoalimenté et permet de démarrer immédiatement la production salivaire.

Ce premier effet obtenu est direct mais, non seulement la pilocarpine accède aux groupes de production de la salive mais aussi, passe pour partie dans la micro vascularisation veineuse sub et per linguale.

Cette partie de la pilocarpine, ainsi absorbée par la muqueuse, rejoint le flot circulatoire systémique.

Dès lors, par la circulation générale, la pilocarpine se retrouve distribuée en quelques minutes aux paquets glandulaires sous-maxillaires, ce qui constitue une seconde action légèrement décalée dans le temps et relevant d'un mécanisme différent.

Cette formulation permet ainsi d'éviter le passage par le foie et la métabolisation associée. La quantité peut être réduite et le rapport dose / effet est fortement augmenté.

De plus, on évite le passage par l'estomac dont le pH altère aussi la pilocarpine.

La pilocarpine provoquant ces deux effets successifs est donc extrêmement avantageuse.

En outre, les effets de stimulation d'après les études de pharmacocinétique connues se prolongent sur une durée de plusieurs heures de l'ordre de 4 à 8 heures.

De fait, quelques milligrammes de pilocarpine, de 2 à 25 mg par jour, pour fixer les idées, sont suffisants en recourant à cette formulation.

La réduction de dose induit une économie potentielle d'effets secondaires. Les seuils de concentration efficace de la composition selon la présente invention sont beaucoup plus bas et souvent inférieurs aux seuils de déclenchement au niveau digestif, cardio-vasculaire, urinaire, et de provocation d'hypersudations, de bouffées de chaleur ou de nausées.

Quant à la pilocarpine restée circulante dans la voie artérielle, nécessairement en très faible quantité, elle rejoint toujours le système hépatique par l'artère hépatique et se trouve définitivement métabolisée. Ceci permet de respecter le métabolisme de détoxification.

Le traitement avec de faibles doses et une formulation parfaitement stable permet aussi de mettre en oeuvre une posologie très modulable, plus précisément adaptée à chaque patient en fonction des exigences souvent très variables, avec des valeurs ajustées à 0,5 mg près.

Une telle formulation autorise ainsi une prise de façon permanente de la pilocarpine car les effets latéraux sont réduits.

Sur le plan de l'action, on comprend que le traitement de stimulation des deux étages de la production salivaire du patient atteint d'hyposialies est très efficace et procure un confort pour le patient.

Ainsi, ce double effet décalé dans le temps de la production immédiate par la sous-muqueuse buccale dès le contact suivie de la stimulation des grosses glandes, parotides, sous-maxillaire, du système salivaire, permet la prise des repas dans de bonnes conditions de réponse salivaire.

## Revendications

1. Comprimé sub-lingual pour le traitement des hyposialies, permettant une dissolution et une fixation locale au niveau des tissus de la sphère bucco-pharyngée, ayant une composition comprenant de la pilocarpine associée à au moins un polymère bioadhésif choisi parmi la famille des dérivés cellulosiques, des gommes, des polymères du type acide alginique et dérivés, macrogols, gélatine, povidone, ou pectines et à un substrat de masse de faible poids moléculaire choisi parmi le sorbitol, le mannitol, le xylitol ou le lactose.

2. Comprimé sub-lingual pour le traitement des hyposialies selon la revendication 1, **caractérisé en ce que** la pilocarpine est la pilocarpine base ou sous forme de sel, chlorhydrate ou nitrate.

3. Comprimé sub-lingual pour le traitement des hyposialies selon l'une quelconque des précédentes revendications, **caractérisé en ce que** la composition comprend au moins un tampon,

4. Comprimé sub-lingual pour le traitement des hyposialies selon l'une quelconque des précédentes revendications, **caractérisé en ce que** la composition comprend au moins un lubrifiant.

5. Comprimé sub-lingual pour le traitement des hyposialies selon l'une quelconque des précédentes revendications, **caractérisé en ce que** la composition comprend au moins un agent adoucissant.

6. Comprimé sub-lingual pour le traitement des hyposialies selon l'une quelconque des précédentes revendications, **caractérisé en ce qu'**il a la formule suivante :
- pilocarpine base ou sous forme de sel : 2,5 mg
- stéarate de magnésium : 10,0 mg
- hydrogénophosphate sodique ou disodique : 90,0 mg
- méthocel K 100 : 50,0 mg
- PolyEthylèneGlycol 6000: 40,0 mg
- acide hyaluronique : 20,0 mg
- lysozyme chlorhydrate : 15,0 mg
- sorbitol qsp comprimé de 1000 mg : 772,5 mg

## Claims

1. Sublingual tablet for the treatment of hypoptyalism, allowing dissolution and local attachment to the tissues of the buccopharyngeal cavity, said sublingual tablet having a composition comprising pilocarpine combined with at least one bioadhesive polymer selected from among the family of cellulose derivatives, gums, polymers of the alginic acid type and derivatives, macrogols, gelatin, povidone, or pectins, and with a mass substrate with a low molecular weight, chosen from sorbitol, manitol, xylitol or lactose.

2. Sublingual tablet for the treatment of hypoptyalism according to claim 1, wherein the pilocarpine is the basic pilocarpine or pilocarpine in the form of salt, chlorohydrate or nitrate.

3. Sublingual tablet for the treatment of hypoptyalism according to any preceding claims, wherein the composition comprises at least a buffer.

4. Sublingual tablet for the treatment of hypoptyalism according to any preceding claims, wherein the composition comprises at least one lubricant.

5. Sublingual tablet for the treatment of hypoptyalism according to any preceding claims, wherein the composition comprises at least one softening agent.

6. Sublingual tablet for the treatment of hypoptyalism according to any preceding claims, wherein it has the following formula:
- pilocarpine that is basic or in salt form: 2.5 mg
- magnesium stearate: 10.0 mg
- sodium or disodium hydrogen phosphate: 90.0 mg
- K 100 methocel: 50.0 mg
- 6 000 polyethylene glycol: 40.0 mg
- hyaluronic acid: 20.0 mg
- lysozyme chlorohydrate: 15.0 mg
- compressed sorbitol qsp for 1000 mg: 772.5 mg

## Patentansprüche

1. Unter der Zunge zu positionierende Tablette zur Behandlung von Hypoptyalismus zum Auflösen und für eine lokale Fixierung auf dem Niveau von Geweben der bukkopharyngealen Sphäre, mit einer Zusammensetzung aufweisend Pilokarpin, das mit zumindest einem bioadhäsiven Polymer verbunden ist, das ausgewählt ist aus der Familie von Zellulosederivaten, Gummis, Polymeren vom Typ Alginatsäure und deren Derivate, Makrogolen, Gelatine, Povidone, oder Pektine und mit einem Substrat geringer molekularer Masse, ausgewählt aus Sorbitol, Manitol, Xylitol oder Laktose.

2. Unter der Zunge zu positionierende Tablette zur Behandlung von Hypoptyalismus nach Anspruch 1, **dadurch gekennzeichnet, dass** das Pilokarpin fundamentales Pilokarpin oder Pilokarpin in Form von Salz, Chlorhydrat oder Nitrat ist.

3. Unter der Zunge zu positionierende Tablette zur Behandlung von Hypoptyalismus nach einem der vorangehenden Ansprüche, **dadurch gekennzeichnet, dass** die Zusammensetzung zumindest einen Tampon umfasst.

4. Unter der Zunge zu positionierende Tablette zur Behandlung von Hypoptyalismus nach einem der vorangehenden Ansprüche, **dadurch gekennzeichnet, dass** die Zusammensetzung zumindest ein Schmiermittel umfasst.

5. Unter der Zunge zu positionierende Tablette zur Behandlung von Hypoptyalismus nach einem der vorangehenden Ansprüche, **dadurch gekennzeichnet, dass** die Zusammensetzung zumindest einen Weichmacher umfasst.

6. Unter der Zunge zu positionierende Tablette zur Behandlung von Hypoptyalismus nach einem der vorangehenden Ansprüche, **dadurch gekennzeichnet, dass** sie folgende Formel aufweist:
- Piokarbin in fundamentaler oder Salzform: 2,5 mg
- Magnesiumstearat: 10,0 mg
- Natrium- oder Dinatriumhydrogenphosphat: 90,0 mg
- Methocel K 100: 50,0 mg
- Polyethylenglykol 6000: 40,0 mg
- Hyaluronansäure: 20,0 mg
- Chlorhydratlysozym: 15,0 mg
- Sorbitol qsp zur Ergänzung auf eine Tablette von 1000 mg: 772,5 mg
